# EUROPÄISCHE PATENTSCHRIFT

(11) **EP 0 564 505 B1**
(45) Veröffentlichungstag und Bekanntmachung des Hinweises auf die Patenterteilung: **21.06.1995**
(21) Anmeldenummer: 92901391.0
(22) Anmeldetag: 23.12.1991
(51) Int. Cl.: A61B 8/00, A61M 5/19, B01F 5/06

(54) **DARREICHUNGSFORM FÜR MIKROBLÄSCHEN-ECHOKONTRASTMITTEL**
DOSAGE FORM FOR MICRO-BUBBLE ECHO CONTRAST AGENTS
FORME D'ADMINISTRATION POUR AGENT CONTRASTANT D'ECHO A MICROBULLES

(30) Priorität: 28.12.1990 CH 4132/90
(43) Veröffentlichungstag der Anmeldung: 13.10.1993
(73) Patentinhaber: BYK GULDEN LOMBERG CHEMISCHE FABRIK GMBH, D-78403 Konstanz (DE)
(72) Erfinder: BELLER, Klaus-Dieter, D-7750 Konstanz 16 (DE)
(86) Internationale Anmeldenummer: EP9102497
(87) Internationale Veröffentlichungsnummer: WO9211928

(56) Entgegenhaltungen:
- EP-A- 0 077 752
- EP-A- 0 148 116
- DE-A- 3 520 772
- DE-A- 3 838 530
- US-A- 4 049 241
- US-A- 4 753 536

## Beschreibung

### Stand der Technik

Es sind zahlreiche als Echokontrastmittel geeignete Zubereitungen bekannt, die oberflächenaktive Stoffe enthalten, die die Bildung von Mikrobläschen (micro bubbles) unterstützen und diese stabilisieren (z.B. EP-A-0077752). Der von den Mikrobläschen reflektierte Ultraschall wird zur Verbesserung der Utraschallbilder von mit Flüssigkeit gefüllten Gefäßen oder Hohlräumen des menschlichen und tierischen Körpers benutzt. Die Mikrobläschen werden erst unmittelbar vor Verabreichung des Kontrastmittels erzeugt.

In der EP-A-0077752 werden verschiedene Methoden zur Erzeugung von Mikrobläschen in Tenside und viskositätserhöhende Substanzen enthaltenden Mischungen angegeben. Es wird vorgeschlagen, diese Mischungen zusammen mit einem Gas mehrmals in eine Spritze aufzuziehen und wieder auszuspritzen. Alternativ wird vorgeschlagen, einen Teil einer Mischung in den restlichen Teil der Mischung, der sich zusammen mit dem Gas in einem Gefäß befindet, einzuspritzen. Bei Verwendung von Kohlendioxid als Gas kann ein Teil der Mischung ein carbonsaures Salz enthalten und der andere Teil der Mischung eine äquivalente Menge einer Säure. Weiterhin wird vorgeschlagen, die Mischung zusammen mit dem Gas unter Überdruck in einem geschlossenen Gefäß aufzubewahren, aus dem kurz vor der Anwendung, nötigenfalls nach Schütteln, der Überdruck abgelassen wird.

Es versteht sich von selbst, daß es auf diese Weise nicht möglich ist, in reproduzierbarer Weise verabreichbare Kontrastmittel herzustellen, die in Mikrobläschen möglichst einheitlicher Größe eine definierte Gasmenge enthalten. Man hat deshalb versucht, das Kontrastmittel in einer Spritze vorzulegen. Vor Gebrauch wird die gewünschte Menge Luft aufgezogen. Dann wird die Spritze über ein Verbindungsstück mit einer leeren zweiten Spritze verbunden. Durch kräftiges Hin- und Herpumpen des Kontrastmittels zwischen den beiden Spritzen werden Mikrobläschen erzeugt. Es zeigte sich jedoch, daß es einer für die Praxis unzumutbaren Sorgfalt bedarf, um einigermaßen brauchbare Suspensionen von Mikrobläschen zu erhalten. Ausserdem ist der erforderliche Kraftaufwand sehr hoch. Eine Standardisierung ist in der Praxis kaum zu erreichen.

Aus der EP-A-0148116 ist eine Vorrichtung zur Homogenisierung von Mikrokapsel-Suspensionen bekannt, die ein Verbindungsstück mit zwei weiblichen Anschlußstutzen für zwei aufzusteckende Spritzen umfaßt. Der Durchgangskanal des Verbindungsstücks weist eine Verengung auf, die die Verwirbelung der Flüssigkeit unterstützen soll, die zwischen den beiden Spritzen hinund hergepumpt wird.

In der DE-A-3838530 wird eine Verpackung für eine Zwei-Komponenten-Masse beschrieben, die aus zwei flexiblen Behältern für die beiden Komponenten besteht. Vor Gebrauch werden die beiden Behälter über einen rohrförmigen Adapter miteinander verbunden, der in seinem Inneren mit zueinander versetzt angeordneten Umlenkwänden versehen ist. Zum Vermischen der Komponenten werden die Inhalte der Behälter mehrmals durch den Adapter hin- und hergepreßt.

Aus der US-A-4,049,241 ist eine Mischkammer für fluide Materialien bekannt, bei der in einem rohrförmigen Gehäuse eine Vielzahl von gegen die Rohrachse geneigten stabförmigen Mischelementen vorgesehen sind.

### Beschreibung der Erfindung

Der Erfindung liegt die Aufgabe zugrunde, eine Darreichungsform für Mikrobläschen-Echokontrastmittel zur Verfügung zu stellen, die es dem Anwender erlaubt, ohne unzumutbaren Aufwand die Mikrobläschen-Echokontrastmittel standardisiert und optimiert zu verabreichen. Ein weiteres Ziel der Erfindung ist es, die Qualität der mit einem Mikrobläschen-Echokontrastmittel erhältlichen Abbildungen von Körperstrukturen zu verbessern und die Reproduzierbarkeit der Bilder zu verbessern.

Ein weiteres Ziel der Erfindung ist die Erhöhung der Anwendungssicherheit durch die Vermeidung des Entstehens von Gasbläschen, die wegen ihres zu großen Durchmessers den Patienten einer Emboliegefahr aussetzen.

Ein weiteres Ziel der Erfindung ist die Vermeidung eines Infektionsrisikos.

Erfindungsgemäß werden diese Aufgaben durch den Gegenstand von Anspruch 1 gelöst. gelöst

Gegenstand der Erfindung ist daher eine Darreichungsform für Mikrobläschen-Echokontrastmittel umfassend eine erste Spritze, welche unlösbar mit einer Mischkammer verbunden ist, die in ihrem Inneren mit Mischelementen versehen ist, welche bei Bedienung der Spritze die Durchwirbelung der gasförmigen und flüssigen Komponenten des Kontrastmittels bewirken, wobei die Mischkammer die für die Herstellung der Mikrobläschen benötigte, vorbestimmte Menge eines geeigneten Sterilgases enthält und durch einen Stopfen oder eine Kappe verschlossen ist.

Bevorzugte Ausführungsformen der Erfindung ergeben sich aus den Patentansprüchen 2-5.

Die Mischkammer ist vorzugsweise rohrförmig und weist in ihrem Innenlumen Mischelemente auf.

In einer bevorzugten Ausführungsform sind die Mischelemente dornartig ausgebildet, d.h. die Mischelemente stehen vorzugsweise senkrecht auf der Innenwand der Mischkammer und zeigen damit in Richtung auf die Rohrlängsachse der Mischkammer. Es ist zweckmäßig, die Mischelemente scharfkantig auszubilden, also Abrißkanten zu schaffen. Vorzugsweise sind die Mischelemente gegeneinander versetzt wendelförmig angeordnet. Zusätzliche Abrißkanten können durch eine oder mehrere Lochblenden erreicht werden.

Echokontrastmittel, die als einheitliche Lösung vorliegen, können in einer Spritze zur Verfügung gestellt, oder vor der Anwendung aus einem Vorratsbehälter, wie z.B. einer Ampulle luftfrei in eine Spritze aufgezogen werden. Sodann wird die Spritze mit dem freien Ende der mit einer Spritze fest verbundenen Mischkammer, deren inneres Volumen die vorbestimmte Menge Gas enthält, konnektiert. Das Echokontrastmittel wird über die Mischkammer in die leere zweite Spritze und anschließend wieder zurück in die erste Spritze gepumpt. Bereits nach wenigen Wiederholungen des Pumpvorganges hat sich eine relativ stabile Mikrobläschen-Suspension gebildet. Das Echokontrastmittel ist nunmehr zum Verabreichen bereit. Der Anwender ersetzt nun die leere Spritze durch eine Injektionsnadel und injiziert das Kontrastmittel. Zweckmäßigerweise erfolgt die Injektion aus der Spritze mit Mischkammer, da in diesem Fall direkt beim Injizieren noch einmal die Mischkammer durchlaufen wird.

Die Herstellung der Spritze mit Mischkammer erfolgt nach dem Fachmann bekannten Verfahren aus für solche medizinischen Artikel üblichen Materialien. Beispielsweise wird die Mischkammer nach dem Spritzgußverfahren hergestellt und mit dem Anschlußstutzen einer gebräuchlichen Spritze unlösbar verbunden, beispielsweise durch Kleben oder Ultraschallverschweißung oder von vornherein einstückig zusammen mit dem Spritzenzylinder hergestellt. Das freie Ende der Mischkammer kann als männliches oder weibliches Anschlußstück gestaltet werden. Für den Fall, daß das freie Ende als männliches Anschlußstück ausgebildet ist, hat die zweite Spritze ein weibliches Anschlußstück, oder es wird auf das männliche Anschlußstück der zweiten Spritze ein Zwischenstück mit zwei weiblichen Anschlüssen gesteckt. Für den Fall, daß das freie Ende als weibliches Anschlußstück ausgebildet ist, wird die Injektionsnadel über ein Zwischenstück mit zwei männlichen Anschlußstücken aufgesteckt.

Es hat sich gezeigt, daß bei Verwendung von Echokontrastmittelzubereitungen, bei denen die Bildung von stabilen Mikrobläschen geeigneter Größe mit relativ geringem Energieaufwand möglich ist, auf die zweite Spritze verzichtet werden kann. In günstigen Fällen reicht bereits das einmalige Durchlaufen der Mischkammer beim Injizieren zur Bildung der Mikrobläschen von ausreichender Qualität und Menge aus. Dieses Vorgehen ist beispielsweise bei Rechtsherzuntersuchungen zweckmäßig.

Die erfindungsgemäße Darreichungsform für Mikrobläschen-Echokontrastmittel wird vorzugsweise als ein für die Anwendung fertiges Set zur Verfügung gestellt.

Besteht das Echokontrastmittel aus einer einzigen flüssigen Komponente, so kann diese z.B. in einer normalen Spritze abgefüllt sein, oder in einer Durchstechflasche vorliegen, aus der es luftfrei in die Spritze aufgezogen wird. Die zu verwirbelnde vorgegebene Menge Gas befindet sich in der Mischkammer. Es hat sich als zweckmäßig herausgestellt, das freie Innenvolumen der Mischkammer so zu wählen, daß es dem Volumen der vorbestimmten Menge an Gas entspricht, die mit den Kontrastmittelkomponenten ein optimales Mikrobläschen-Echokontrastmittel ergibt. Die Anschlußstutzen der beiden Spritzen werden mit entsprechenden Kappen bzw. Stopfen verschlossen. Es ist zweckmäßig, die genannten Teile zusammen mit einer Injektionsnadel und gewünschtenfalls mit weiteren bei i.V.-Anwendungen üblichen Hilfsmitteln wie Alkoholtupfer und Verbandpflaster steril in einer Verpackung, beispielsweise einer üblichen Blisterpackung, bereitzustellen.

Als Kontrastmittelkomponenten eignen sich solche Zusammensetzungen, die beim Aufschäumen mit Gasen für Ultraschalluntersuchungen genügend stabile Mikrobläschen ergeben. Die Komponenten enthalten oberflächenaktive Stoffe und gewünschtenfalls weitere die Stabilisierung von Mikrobläschen fördernde Stoffe, wie z.B. viskositätserhöhende Stoffe. Geeignete Komponenten sind beispielsweise in EP-A-0 077 752 und EP-A-0 212 568 beschrieben.
gemäß einer besonderen Ausführungsform der Erfindung enthält die Darreichungsform für Mikrobläschen-Echokontrastmittel 1 bis 20 ml, vorzugsweise 2 bis 8 ml, und besonders bevorzugt 5 ml flüssige Kontrastmittelkomponente. Pro 1 ml flüssige Kontrastmittelkomponente sind dann 0,01 bis 0,1, vorzugsweise 0,04 bis 0,06 ml Gas enthalten.

Eine bevorzugte Ausführung der erfindungsgemäßen Darreichungsform umfaßt zwei Spritzen von 5 ml Innenvolumen, von denen die eine unlösbar mit einer Mischkammer, die ein Innenvolumen von 0,18 ml aufweist, verbunden ist. Die mit der Mischkammer unlösbar verbundene Spritze ist leer. Die Mischkammer enthält 0,18 ml Gas, vorzugsweise sterile Luft und ist entweder durch einen Stopfen oder eine Kappe verschlossen. In der zweiten Spritze befinden sich 3 ml einer flüssigen Kontrastmittelkomponente. Auch diese Spritze ist durch einen Stopfen oder Kappe verschlossen.

Nachstehend soll die Erfindung anhand der Figuren 1 bis 4 näher erläutert werden.
- Fig. 1: zeigt einen Längsschnitt durch eine Mischkammer.
- Fig. 2: zeigt einen Querschnitt durch eine Mischkammer längs der Linie II in Fig. 1.
- Fig. 3: zeigt eine Seitenansicht einer Spritze mit Mischkammer.
- Fig. 4: zeigt eine Seitenansicht einer Spritze mit Mischkammer mit aufgesetzter zweiter Spritze.

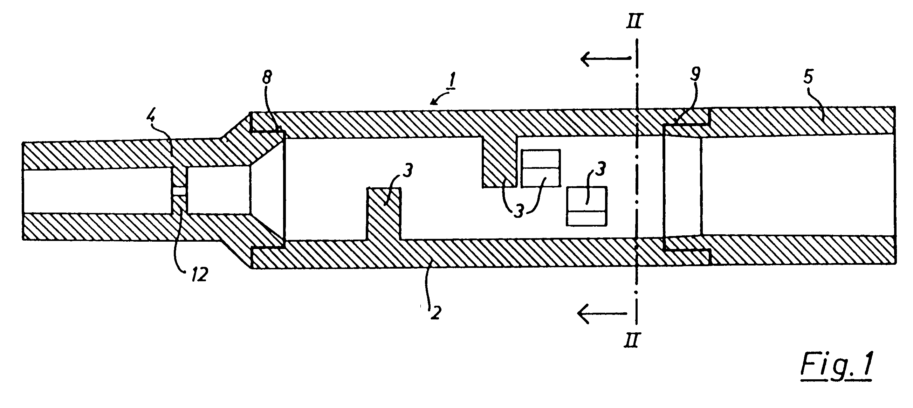

In Fig. 1 ist eine Ausführungsform der Mischkammer 1 im Längsschnitt dargestellt. Die zylindrische Rohrhülse 2 weist senkrecht auf ihrer Innenwand stehende Mischelemente 3 auf, die gegeneinander versetzt wendelartig oder spiralig angeordnet sind. Am linken Ende ist ein Anschlußstutzen 4 angeformt, der als männlicher Lueranschluß ausgebildet ist. Am rechten Ende ist ein Anschlußstutzen 5 angeformt, der als weiblicher Lueranschluß ausgebildet ist. Aus herstellungstechnischen Gründen erscheint es zweckmäßig, die Mischkammer aus drei Teilen, d.h. aus der Rohrhülse 2 mit den Mischelementen 3 und den beiden Anschlußstutzen 4, 5 zu fertigen und auf geeignete Weise zu verbinden. Die Verbindungslinien sind in Fig. 1 mit 8 und 9 bezeichnet. Anstelle des Anschlußstückes 5 kann direkt ein entsprechendes Anschlußteil einer Kolbenspritze stehen. Wie weiter oben ausgeführt, kann der Anschlußstutzen 4 auch als weiblicher Lueranschluß gestaltet sein. Gewünschtenfalls ist im Anschlußstutzen 4 eine Lochblende 12 vorgesehen. Durch diese fakultative Lochblende 12 kann eine Intensivierung der Verwirbelung erreicht werden.

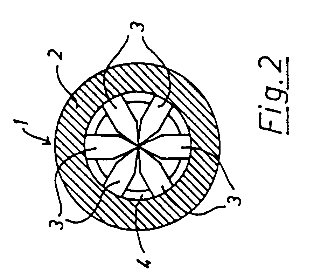

Fig. 2 zeigt einen Querschnitt durch eine Mischkammer entlang der Linie 2 in Fig. 1. Man erkennt die dornartig senkrecht auf der Innenwand der Rohrhülse 2 stehenden Mischelemente 3.

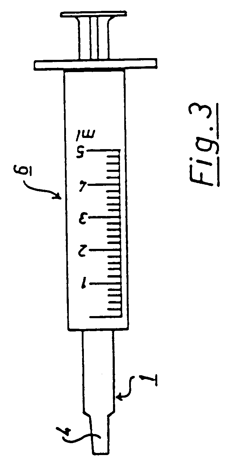

In Fig. 3 ist eine Spritze 6 mit Mischkammer 1 mit männlichem Anschlußstück dargestellt, wobei die Mischkammer 1 direkt am Boden des Spritzenzylinders angeformt ist.

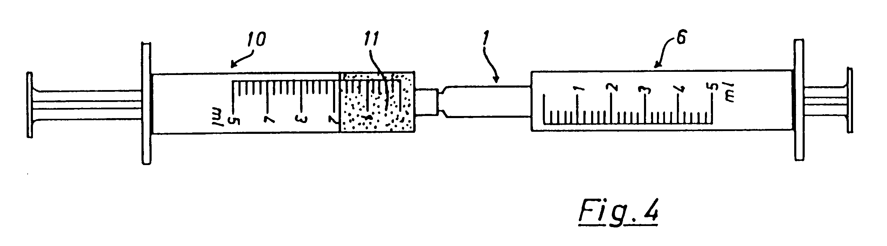

Fig. 4 zeigt die Spritze 6 mit angeformter Mischkammer 1, auf deren männlichem Anschlußstutzen eine zweite Spritze 10 aufgesteckt ist, die das Echokontrastmittel 11 enthält. Die für die Mikrobläschen benötigte Gasmenge ist in Mischkammer 1 enthalten. Durch Hin- und Herpumpen des Echokontrastmittels 11 zwischen Spritze 10 und Spritze 6 über die Mischkammer 1 wird die zur Verabreichung fertige Mikrobläschen-Suspension bereitet.

Durch die erfindungsgemäße Darreichungsform erreicht man eine erhöhte Anwendungssicherheit. Hier ist vor allem hervorzuheben, daß durch die vorbestimmte Menge Gas genau die Gasmenge zur Anwendung kommt, die optimal an die Zusammensetzung und Menge der flüssigen Komponente angepaßt ist und damit zu einer maximalen Aussagekraft der erhaltbaren Bilder führt. Fehler, die durch herkömmliches Aufziehen von zu viel oder zu wenig Gas entstehen können, werden dadurch sicher vermieden. Insbesondere das Aufziehen von zu viel Gas könnte zu einer Emboliegefahr für den Patienten führen. Aber auch das Aufziehen von zu wenig Gas nach der herkömmlichen Methode kann wegen mangelhafter Bildgebung die Wiederholung der Untersuchung notwendig machen. Außerdem ist durch die erfindungsgemäße Darreichungsform die Infektionsgefahr für den Patienten erheblich reduziert, da im Gegensatz zu dem bisher üblichen Aufziehen von Umgebungsluft steriles Gas verwendet wird und nicht eventuell mit resistenten Krankenhauskeimen verseuchte Umgebungsluft über die Mikrobläschen in den Blutkreislauf des Patienten gelangt. Hierzu kommt, daß durch die unlösbare Verbindung zwischen Mischkammer und Spritze im Hinblick auf eine mögliche Keimeinschleichung Konnektierungsvorgänge vermieden werden.

Die Anwendungssicherheit wird auch dadurch stark verbessert, daß es nunmehr möglich ist, die Zahl der Hin- und Herpumpvorgänge erheblich zu verringern, wobei gleichzeitig die Qualität der Mikrobläschen-Suspension ansteigt. Da außerdem der für das Hin- und Herpumpen notwendige Kraftaufwand erheblich vermindert wird, steigt die Bereitschaft des Anwenders, den Aufschäumvorgang vorschriftsmäßig bis zum Erreichen einer für den angestrebten Zweck optimalen Beschaffenheit des Mikrobläschen-Echokontrastmittels durchzuführen.

Die erfindungsgemäße Darreichungsform führt zu Mikrobläschen-Suspensionen, die sich durch eine überraschende verbesserte Kontrastqualität auszeichnen.

Außerdem wurde beobachtet, daß nach Verschwinden des Kontrastes aus dem Lumen der untersuchten Körperhohlräume die inneren Oberflächen der Körperhöhlen für eine geraume Zeit überraschend gut sichtbar bleiben. Beispielsweise eignet sich diese bisher nur mit den neuen Darreichungsformen beobachtete Zeichnung von Oberflächen hervorragend zur Diagnose des Herzens. Anscheinend begünstigt die neue Darreichungsform die Entstehung von an inneren Körperoberflächen haftenden Mikrobläschen.

In einem Vergleichsversuch wurden jeweils 3 ml einer vernetzte Polypeptide enthaltenden Infusionslösung (Haemaccel ® der Fa. Behringwerke), die sich zur Herstellung von Mikrobläschen-Kontrastmitteln eignet, zusammen mit 0,18 ml Luft zum einen fünfmal zwischen zwei über einen Dreiwegehahn verbundene Spritzen (Versuch A) und zum anderen fünfmal in einer erfindungsgemäßen Zubereitungsform zwischen der mit einer Mischkammer verbundenen Spritze und einer auf die Mischkammer aufgesetzten Spritze (Versuch B) hin- und hergepumpt. Anschließend wurden die Zubereitungen A und B wachen Hunden i.v. verabreicht. Eine Echokardiographie des rechten Ventrikels wurde mit einem Sonoscope 4 bei 3,5 MHz durchgeführt. Die erhaltenen Videoausdrucke wurden densitometrisch ausgewertet. Das Densitometer (Gretag D182) mißt Änderungen der Helligkeit (brightness) im Bereich von 0,00 bis 2,50 DU (Density units) in 100 Schritten. Die Kalibrierung erfolgt anhand von Kalibrierungskarten (calibration references), wobei das hellste Weiß dem Wert 0,00 und das dunkelste Schwarz dem Wert 1,64 entspricht. Der Wert für jedes Tier wird aus dem Mittel von vier einzelnen Messungen innerhalb eines Quadratzentimeters ermittelt.

Es wurden folgende Ergebnisse erhalten:

| | Kontrastqualität | maximale Intensität | 10 sec p.A. | 20 sec p.A. |
|---|---|---|---|---|
| Versuch A | grob | 1,06 | 0,39 | 0,19 |
| Versuch B | fein | 1,56 | 1,09 | 0,58 |

Es zeigt sich, daß die erfindungsgemäße Darreichungsform zusätzlich zu der einfacheren und sichereren Anwendung nicht nur zu einer erheblich besseren Kontrastqualität, sondern auch zu einer überraschenden Intensitätserhöhung führt, wobei der qualitativ und quantitativ überlegene Kontrast sogar noch sehr viel länger beobachtbar ist.

Durch die neue Darreichungsform für Mikrobläschen-Echokontrastmittel wird nicht nur die Handhabung von Echokontrastmitteln vereinfacht und die Anwendungssicherheit erhöht, sondern vor allem ein erheblicher zusätzlicher diagnostischer Erkenntnisgewinn erzielt.

## Patentansprüche

1. Darreichungsform für Mikrobläschen-Echokontrastmittel umfassend eine erste Spritze (6), welche unlösbar mit einer Mischkammer (1) verbunden ist, die in ihrem Inneren mit Mischelementen (3) versehen ist, welche bei Bedienung der Spritze (6) die Durchwirbelung der gasförmigen und flüssigen Komponenten des Kontrastmittels bewirken, wobei die Mischkammer (1) die für die Herstellung der Mikrobläschen benötigte, vorbestimmte Menge eines geeigneten Sterilgases enthält und durch einen Stopfen oder eine Kappe verschlossen ist.

2. Darreichungsform nach Anspruch 1, umfassend zusätzlich eine zweite Spritze (10).

3. Darreichungsform nach Anspruch 2, dadurch gekennzeichnet, daß die zweite Spritze (10) die flüssige Komponente des Kontrastmittels enthält.

4. Darreichungsform nach Anspruch 2, umfassend zusätzlich eine Durchstechflasche enthaltend die flüssige Komponente des Kontrastmittels.

5. Darreichungsform nach Anspruch 1, dadurch gekennzeichnet, daß sie als ein für die Anwendung fertiges Set vorliegt.

## Claims

1. Dosage form for micro bubble echo contrast media comprising a first syringe (6) which is unreleasably connected to a mixing chamber (1) which is provided in its interior with mixing elements (3) which, on operation of the syringe (6), bring about turbulence of the gaseous and liquid components of the contrast medium, the mixing chamber (1) containing the predetermined amount, which is needed to produce the micro bubbles, of a suitable sterile gas and being closed by a stopper or cap.

2. Dosage form according to Claim 1, additionally comprising a second syringe (10).

3. Dosage form according to Claim 2, characterised in that the second syringe (10) contains the liquid contrast medium component.

4. Dosage form according to Claim 2, additionally comprising a vial containing the liquid contrast medium component.

5. Dosage form according to Claim 1, characterised in that it is in the form of a set ready for use.

## Revendications

1. Forme d'administration pour des agents d'écho-contraste à microbulles, comprenant une seringue (6) qui est reliée de manière inamovible à une chambre de mélange (1), l'intérieur de ladite chambre étant pourvu d' éléments de mélange (3), qui assurent, lors de la mise en service de la seringue (6), un tourbillonnement ou une fluidisation intime des composants gazeux et liquides de l'agent de contraste, où la chambre de mélange (1) contient une quantité prédéterminée d'un gaz stérile approprié, nécessaire à la formation des microbulles et est fermée par un bouchon ou une coiffe ou capsule.

2. Forme d'administration suivant la revendication 1, comprenant complémentairement une seconde seringue (10).

3. Forme d'administration suivant la revendication 2, caractérisée en ce que la seconde seringue (10) contient le composant liquide de l'agent de contraste.

4. Forme d'administration suivant la revendication 2, comprenant complémentairement un flacon à percer qui contient le composant liquide de l'agent de contraste.

5. Forme d'administration suivant la revendication 1, caractérisée en ce qu'elle se présente sous forme d'une trousse prête à l'emploi.
